# EUROPEAN PATENT APPLICATION

(11) **EP 4 253 557 A1**
(43) Date of publication of application: **04.10.2023**
(21) Application number: 22894249.6
(22) Date of filing: 23.06.2022
(51) Int. Cl.: C12Q 1/68, C12N 15/11

(54) **PRIMER/PROBE DESIGN METHOD, DETECTION COMPOSITION AND KIT FOR MIRNA DETECTION**

(30) Priority: 16.11.2021 CN 202111357909
(71) Applicant: Sansure Biotech Inc., Changsha, Hunan 410205 (CN)
(72) Inventor: DAI, Lizhong, Changsha, Hunan 410205 (CN); WANG, Pinyi, Changsha, Hunan 410205 (CN); DING, Pinghui, Changsha, Hunan 410205 (CN); JI, Bozhi, Changsha, Hunan 410205 (CN); GUO, Jun, Changsha, Hunan 410205 (CN); ZHOU, Rong, Changsha, Hunan 410205 (CN); LIU, Jia, Changsha, Hunan 410205 (CN)
(74) Representative: Novagraaf Technologies
(86) International application number: PCT/CN2022/100692
(87) International publication number: WO 2023/087706

(57) **Abstract**

The disclosure belongs to the field of molecular biological detection, particularly belongs to the field of miRNA detection, and more particularly relates to one-step detection of miRNA. The disclosure provides a primer and probe design method for miRNA detection, a detection composition including a primer and probe designed by the primer and probe design method, and a detection kit including the detection composition. By using the primer and the probe designed by the method disclosed in the disclosure, miRNA detection may be carried out by adopting one-step method, and non-specific amplification is avoided; the method has a high sensitivity of 1000 copies/mL with good specificity, and the operation of the method is simpler and safer with more credible results.

## Description

### CROSS-REFERENCE TO RELATED APPLICATIONS

This disclosure is a continuation of International Application No. PCT/CN2022/100692, filed on June 23, 2022, which claims priority to Chinese patent application CN202111357909.9 filed on November 16, 2021. All of the aforementioned patent applications are hereby incorporated by reference in their entireties.

### TECHNICAL FIELD

The disclosure belongs to the field of molecular biological detection, in particular, to the field of miRNA detection, and more specifically, to one-step detection of miRNA.

### BACKGROUND

MicroRNAs (miRNAs) are endogenous single-stranded non-coding small RNAs consisting of 20-25 bases. MicroRNAs regulate a variety of biological processes, including growth and development, signal transduction, immune regulation, apoptosis, proliferation, and tumorigenesis. Due to the powerful regulatory functions of miRNAs, their abnormal expression levels are considered biomarkers for a variety of diseases, including neurodegenerative diseases (and central nervous system damage), diabetes, cardiovascular disease, kidney disease, liver disease, and even immunity dysfunction. miRNAs have specific expression profiles in cancer cells and tissues and can enter the humoral circulation. Circulating free miRNAs may be detected in serum, plasma, and other body fluids. Mature miRNAs are very stable in body fluids. This makes miRNAs potential non-invasive tumor markers for human cancers. Currently, there are various methods to detect miRNA, including RNA high-throughput sequencing, microchip and RT-qPCR, and so on. The advantages of sequencing and microarray methods lie in high-throughput miRNA analysis, discovering novel miRNAs, or finding differentially expressed miRNAs associated with diseases, but they are not suitable for routine use. For the diagnostic field, the most promising method is still the gold standard RT-qPCR. At present, the commonly used RT-qPCR methods are stem-loop reverse transcription method and ployA tailing method.

However, the relevant scientific research kits for miRNA detection are still clinical detection products, and both reverse transcription and qPCR amplification are performed step by step. Although RNA one-step amplification is widely used in clinical detection, miRNA one-step amplification is not popular mainly because its fragments are too small (20nt-25nt). When using conventional RNA one-step amplification, the non-specific amplification will be amplified; moreover, the circulating miRNA content is inherently low, and one-step amplification will lose sensitivity. Therefore, the two-step method is still the first choice for miRNA detection. However, the long operation time of two-step amplification, and the increasing possibility of contamination by opening the lid and pipetting, cannot meet the clinical needs.

Therefore, there is a need for one-step detection of miRNA, which does not need to open the lid midway, so as to reduce contamination and operation time, and simultaneously ensure the detection sensitivity

### SUMMARY

In view of this, in a first aspect, the main object of the disclosure is to provide a primer and probe design method for miRNA detection, including the following steps:
S1: designing a forward primer, wherein the sequence of the forward primer is exactly the same as a sequence obtained by deleting the last N bases from the 3' end of a target miRNA and replacing U in the sequence with T so that the Tm value of the forward primer is 56-60°C;
S2: designing a stem-loop primer, wherein the forward primer is compared with a universal stem-loop primer to find out a region in which the number of consecutive bases of complementary pairing is greater than 5, then base substitution or deletion is conducted in the sequence of the universal stem-loop primer so that the number of consecutive bases of complementary pairing is less than or equal to 5, or the dG between the stem-loop primer and the forward primer is >-8kc/m;
S3: designing a reverse primer; and
S4: designing a probe;
wherein N is 5-8.

By using the primer and the probe designed by the method disclosed in the invention, miRNA detection may be carried out by adopting a one-step method, and non-specific amplification is avoided; the method has a high sensitivity of 1000 copies/mL with good specificity, and the operation of the method is simpler and safer with more credible results.

In some particular embodiments, step S2 is designing a stem-loop primer, wherein the forward primer is compared with a universal stem-loop primer to find out a region in which the number of consecutive bases of complementary pairing is greater than 5, then base substitution or deletion is conducted in the sequence of the universal stem-loop primer so that the number of consecutive bases of complementary pairing is less than or equal to 5.

In some particular embodiments, step S2 is designing a stem-loop primer, wherein the forward primer is compared with a universal stem-loop primer to find out a region in which the number of consecutive bases of complementary pairing is greater than 5, then base substitution or deletion is conducted in the sequence of the universal stem-loop primer so that the dG between the stem-loop primer and the forward primer is >-8kc/m.

Further, as known to those skilled in the art, for the modified stem-loop primer of the disclosure, the integrity of the stem-loop structure may be determined by using relevant software.

Further, a reverse primer is designed, wherein this step includes making the reverse primer sequence identical to a part of the stem-loop primer, and the number of consecutive bases of complementary pairing for the stem-loop primer is less than or equal to 5 so that the Tm value of the reverse primer is 56-60°C

Further, the starting point of the reverse primer is located at the starting point of the circular sequence of the stem-loop primer, or 1-8 bp deviation to the 5' end, or 1-3 bp deviation to the 3' end from the starting point of the circular sequence.

In the disclosure, the "starting point of the reverse primer" refers to the starting point on the template (that is the stem-loop primer) for designing the reverse primer.

By placing the starting point of the reverse primer at or near the circular sequence of the stem-loop sequence, the sensitivity of miRNA detection is higher.

Further, a probe is designed, wherein this step comprises making the probe sequence include two sequence segments F and G from the 5' end, the first sequence segment F is identical to the 3' end sequence of the stem-loop primer, and the second segment G is identical to the complementary sequence of the last N bases at the 3' end of the target miRNA so that the Tm value of the probe is 68-72°C.

Further, the 5' end of the probe is connected to a fluorescent group, and the 3' end is connected to a quenching group. Preferably, the first base of the probe cannot be a G.

In the disclosure, the fluorescent group may be selected from the group consisting of FAM, HEX, ROX, VIC, CY5, 5-TAMRA, TET, CY3, and JOE, but is not limited thereto. The quenching group may be selected from, but not limited to, MGB, BHQ1, or BHQ2.

In some particular embodiments, the size of the forward primer is 18-28 bp.

In some particular embodiments, the size of the stem-loop primer is 50-60 bp.

In some particular embodiments, the size of the reverse primer is 18-28 bp.

In some particular embodiments, the size of the probe is 13-20 bp.

In a particular embodiment, in order to make the Tm value of the forward primer 56-60°C, a random sequence is designed in front of the forward primer, so that the Tm value of the forward primer is 56-60°C.

In some particular embodiments, the sequences of the universal stem-loop sequences are set forth in SEQ ID NOs: 1-4.

In a preferred embodiment, N is 6.

When N is 6, a primer probe thus designed has higher sensitivity for miRNA detection, and the Ct value is further advanced.

In some particular embodiments, all primers and probes cannot overlap each other.

In a second aspect, the disclosure provides a miRNA detection composition including the primers and probes designed by the above method.

In a particular embodiment, each component of the miRNA detection composition of the disclosure is present in the same package.

In a particular embodiment, the miRNA detection composition of the disclosure is present in the same package in a mixed form.

In a third aspect, the disclosure provides the use of the above-mentioned miRNA detection composition in the preparation of a kit for detecting miRNA

In a fourth aspect, the disclosure provides a miRNA detection kit, which includes the above-mentioned miRNA detection composition.

In some particular embodiments, the above detection kit further includes a PCR amplification system.

The miRNA detection kit further includes 100-200 mM Tris-HCl, 30-100 mM (NH₄)₂SO₄, 200-300 mM KCl, 0.2-0.8% Tween-20, 0.02-0.08% gelatin, 0.05-0.2% BSA, 5-20 mM EDTA, 2-10 M betaine, 5-15% sorbitol, 5-20% mannitol, 15-30 µg/µl gp32, 0.2-0.3M TMAC, 1-5% glycerol, and 5-15% PEG 6000.

In a particular embodiment, the miRNA detection kit further includes 150 mM Tris-HCl, 50 mM (NH₄)₂SO₄, 250 mM KCl, 0.5% Tween-20, 0.05% gelatin, 0.1% BSA, 10 mM EDTA, 5M betaine, 10% sorbitol, 15% mannitol, 25 µg/µl gp32, 0.25 M TMAC (Tetramethylammonium chloride), 3% glycerol, and 10% PEG 6000.

In some particular embodiments, the above miRNA detection kit further includes at least one of a nucleic acid preservation agent or a nucleic acid release agent.

By combining the PCR system with the primers and probes designed by the method according to the disclosure, the Ct value may be further advanced (that is, the sensitivity is higher).

Further, the PCR system includes dNTP, Mg²⁺, reverse transcriptase, polymerase, and Rnasin.

In a fifth aspect, the disclosure provides a method for one-step detection of miRNA, including the following steps:
1) releasing the nucleic acid of a sample to be tested;
2) detecting the nucleic acid obtained in the above step 1) by using the above composition or the above kit;
3) obtaining and analyzing the results.

Further, performing the detection includes performing reverse transcription and then performing fluorescence quantitative PCR.

Furthermore, reverse transcription and fluorescence quantitative PCR are performed in one tube.

Furthermore, the samples to be tested are serum, plasma, and other body fluids. Preferably, the sample to be tested is serum or plasma.

The disclosure provides a non-diagnostic method for one-step detection of miRNA, the method includes the following steps:
1) releasing the nucleic acid of a sample to be tested;
2) detecting the nucleic acid obtained in the above step 1) by using the above composition or the above kit;
3) obtaining and analyzing the results.

Further, performing the detection comprises performing reverse transcription and then performing fluorescence quantitative PCR.

Furthermore, reverse transcription and fluorescence quantitative PCR are performed in one tube.

### BRIEF DESCRIPTION OF THE DRAWINGS

Fig. 1 is a graph showing one-step amplification of miRNA by stem-loop reverse transcription;
Fig. 2 is the results of comparing one-step detection on specific stem-loop primers and conventional stem-loop primers for target miRNA hsa-miR-106a;
Fig. 3 is the results of comparing one-step detection on specific stem-loop primers and conventional stem-loop primers for target miRNA hsa-miR-let7a;
Fig. 4 is the results of comparing one-step detection on specific stem-loop primers and conventional stem-loop primers for target miRNA hsa-miR-26b;
Fig. 5 is the results of comparing one-step detection on specific stem-loop primers and conventional stem-loop primers for target miRNA hsa-miR-92a-3p;
Fig. 6 shows the sensitivity of the detection method of the disclosure;
Fig. 7 shows the specificity of the detection method of the disclosure;
Fig. 8 is a graph of the impact of the reverse primer miR-106a-SL-R designed in the disclosure on the sensitivity of miRNA one-step detection;
Fig. 9 is a graph of the impact of the reverse primer miR-106a-SL-Ra designed in the disclosure on the sensitivity of miRNA one-step detection;
Fig. 10 is a graph of the impact of the reverse primer miR-106a-SL-Rb designed in the disclosure on the sensitivity of miRNA one-step detection;
Fig. 11 is a graph of the impact of the reverse primer miR-106a-SL-Rc designed in the disclosure on the sensitivity of miRNA one-step detection;
Fig. 12. is a graph showing the impact of the forward primers designed when N is 5 in the disclosure on the sensitivity of miRNA one-step detection;
Fig. 13. is a graph showing the impact of the forward primers designed when N is 6 in the disclosure on the sensitivity of miRNA one-step detection;
Fig. 14. is a graph showing the impact of the forward primers designed when N is 7 in the disclosure on the sensitivity of miRNA one-step detection;
Fig. 15. is a graph showing the impact of the forward primers designed when N is 9 in the disclosure on the sensitivity of miRNA one-step detection.

### DETAILED DESCRIPTIONS OF THE EMBODIMENTS

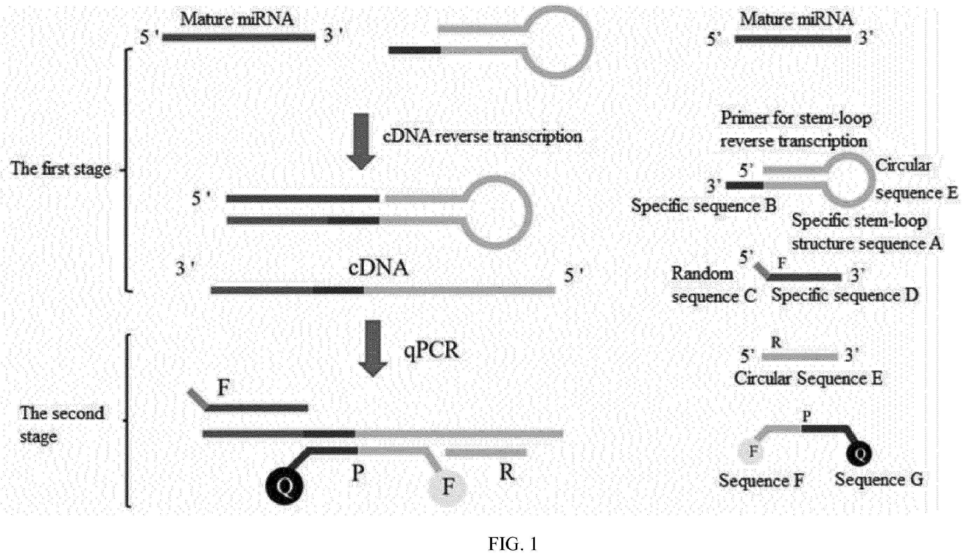

The specific steps of miRNA detection by stem-loop reverse transcription are shown in Fig. 1. Particularly, it is divided into two stages: the first stage, the reverse transcription stage, in which the stem-loop primers are bound to the target miRNA to perform reverse transcription to obtain cDNA; the second stage, amplification detection stage, in which fluorescent PCR is performed by using the forward primer, reverse primer and probe to detect miRNA.

In the disclosure, the term "universal stem-loop sequence" refers to all sequences consisting of the part of the stem-loop structure composed of the stem sequence and the circular sequence, and the part of the specific target miRNA, which may be used for miRNA detection.

In the disclosure, the term "target miRNA" refers to the microRNA to be detected.

In the disclosure, the term "forward primer" refers to a primer used to amplify the 5' end of the cDNA obtained by reverse transcription of the target miRNA.

In the disclosure, the term "reverse primer" refers to a primer used to amplify the 3' end of the cDNA obtained by reverse transcription of the target miRNA.

In the disclosure, the term "two-step method" means that in the process of miRNA detection, the reverse transcription stage and the amplification detection stage are divided into the following steps: firstly adding stem-loop primers for reverse transcription to obtain cDNA, then adding the forward primer, reverse primer and probe to amplify the cDNA and then detect it.

In the disclosure, the term "one-step method" means that in the process of miRNA detection, the reverse transcription stage and the amplification detection stage are performed simultaneously in one tube, that is, the stem-loop primer, forward primer, reverse primer and probe are simultaneously added to directly perform reverse transcription and then perform amplification detection.

### Example 1: Design of primers and probes for miRNA one-step detection

The nucleotide sequences hsa-miR-106a, hsa-miR-let7a, hsa-miR-26b, and hsa-miR-92a-3p disclosed in the micro RNA database (as shown in Table 1) were used as templates, and the specific primers (forward F, reverse R, stem-loop RT) and probe (P) sequences were designed according to the method of the disclosure, particularly the primers and probe sequences are shown in Table 2 below. At the same time, four conventional miRNA primers (forward F, reverse R, stem-loop RT) and probe (P) sequences were designed for comparison, as shown in Table 3.

**Table 1: Four miRNA sequences**

| miRNA | Sequences | SEQ ID NO: |
|---|---|---|
| hsa-miR-106a | AAAAGUGCUUACAGUGCAGGUAG | 5 |
| hsa-miR-let7a | UGAGGUAGUAGGUUGUAUAGUU | 6 |
| hsa-miR-26b | UUCAAGUAAUUCAGGAUAGGU | 7 |
| hsa-miR-92a-3p | UAUUGCACUUGUCCCGGCCUGU | 8 |

**Table 2: Specific stem-loop primers and probe sequences**

| miRNA to be detected | Names of primers or probes | Nucleotide sequences 5'-3' | SEQ ID NO: |
|---|---|---|---|
| hsa-miR-106a | miR-106a-SL-F | GCGTCAAAAGTGCTTACAGTGC | 9 |
| hsa-miR-106a | miR-106a-SL-R | GTAGCGTGGAGTCGGCAATT | 10 |
| hsa-miR-106a | miR-106a-SL-P | ATCGGATACGACCTACCT | 11 |
| hsa-miR-106a | miR-106a-SL-RT | GTCGTATCCGATCGTAGCGTGG AGTCGGCAATTGATCGGATACG ACCTACCT | 12 |
| hsa-miR-let-7a | miR-let7a-SL-F | GCGGCTGAGGTAGTAGGTTGT | 13 |
| hsa-miR-let-7a | miR-let7a-SL-R | GAAGGCTGGAGAGCGAGAT | 14 |
| hsa-miR-let-7a | miR-let7a-SL-P | TACGACTACGATAACTAT | 15 |
| hsa-miR-let-7a | miR-let7a-SL-RT | ATCGTAGTCGTACGAAGGCTGG AGAGCGAGATGTACGACTACG ATAACTAT | 16 |
| hsa-miR-26b | miR-26b-SL-F | TCGCCATTCAAGTAATTCAGG | 17 |
| hsa-miR-26b | miR-26b-SL-R | CTCACGCTCGAGACGGATC | 18 |
| hsa-miR-26b | miR-26b-SL-P | CGAGCAATAGCTCACCTAT | 19 |
| hsa-miR-26b | miR-26b-SL-RT | GAGCTATTGCTCGCCTCACGCT CGAGACGGATCTGGCGAGCAA TAGCTCACCTAT | 20 |
| hsa-miR-92a-3p | miR-92a-SL-F | CGCTATTGCACTTGTCCCG | 21 |
| hsa-miR-92a-3p | miR-92a-SL-R | CGAGAGCTGCTCACGGAGAT | 22 |
| hsa-miR-92a-3p | miR-92a-SL-P | CATCCTCATGCTACAGGC | 23 |
| hsa-miR-92a-3p | miR-92a-SL-RT | AGCATGAGGATGCGAGAGCTG CTCACGGAGATGCATCCTCATG CTACAGGC | 24 |

**Table 3: Conventional universal stem-loop primers and probe sequences**

| miRNA to be detected | Names of primers or probes | Nucleotide sequences 5'-3' | SEQ ID NO: |
|---|---|---|---|
| hsa-miR-106a | miR-106a-L-F | GCCGAGAAAAGTGCTTACAGT GC | 25 |
| hsa-miR-106a | miR-106a-L-R | GTGTCGTGGAGTCGGCAATT | 26 |
| hsa-miR-106a | miR-106a-L-P | CACTGGATACGACCTACCT | 27 |
| hsa-miR-106a | miR-106a-L-RT | GTCGTATCCAGTGCGTGTCGTG GAGTCGGCAATTGCACTGGATA CGACCTACCT | 28 |
| hsa-miR-let-7a | miR-let7a-L-F | CGGCAGGTGAGGTAGTAGGTT GT | 29 |
| hsa-miR-let-7a | miR-let7a-L-R | AGTGCAGGGTCCGAGGTATTC | 30 |
| hsa-miR-let-7a | miR-let7a-L-P | ACTGGATACGACAACTAT | 31 |
| hsa-miR-let-7a | miR-let7a-L-RT | GTCGTATCCAGTGCAGGGTCCG AGGTATTCGCACTGGATACGAC AACTAT | 32 |
| hsa-miR-26b | miR-26b-L-F | CCGCCATTCAAGTAATTCAGG | 33 |
| hsa-miR-26b | miR-26b-L-R | AGTGCAGGGTCCGAGGTATTC | 34 |
| hsa-miR-26b | miR-26b-L-P | CACTGGATACGACACCTAT | 35 |
| hsa-miR-26b | miR-26b-L-RT | GTCGTATCCAGTGCAGGGTCCG AGGTATTCGCACTGGATACGAC ACCTAT | 36 |
| hsa-miR-92a-3p | miR-92a-L-F | CCGTATTGCACTTGTCCCG | 37 |
| hsa-miR-92a-3p | miR-92a-L-R | GTGTCGTGGAGTCGGCAATT | 38 |
| hsa-miR-92a-3p | miR-92a-L-P | ACTGGATACGACACAGGC | 39 |
| hsa-miR-92a-3p | miR-92a-L-RT | GTCGTATCCAGTGCGTGTCGTG GAGTCGGCAATTGCACTGGATA CGACACAGGC | 40 |

The 5' end of the probe is fluorescently labeled, and the 3' end is labeled with MGB.

### Example 2: miRNA one-step detection

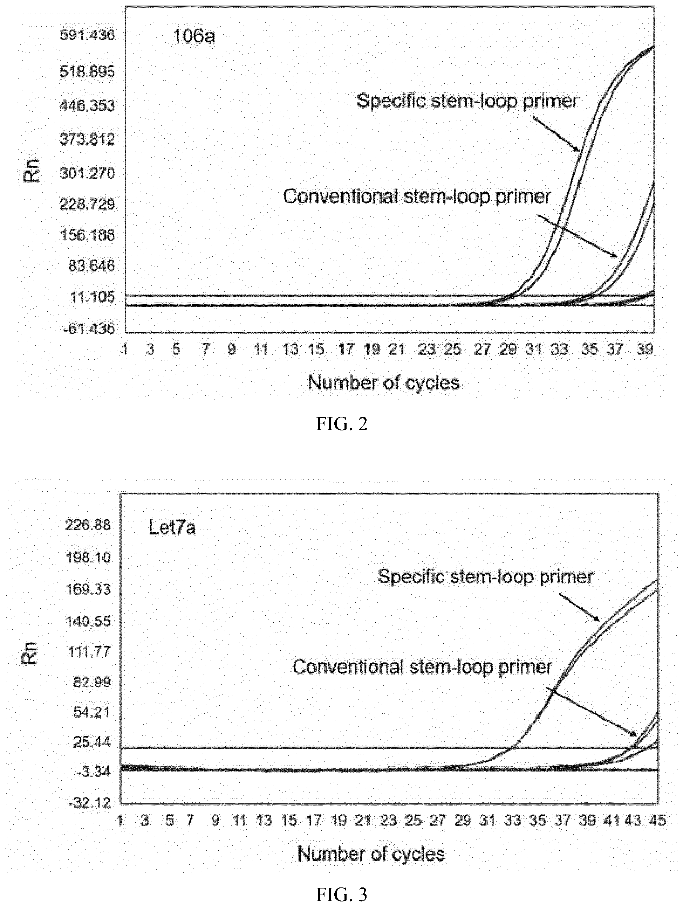

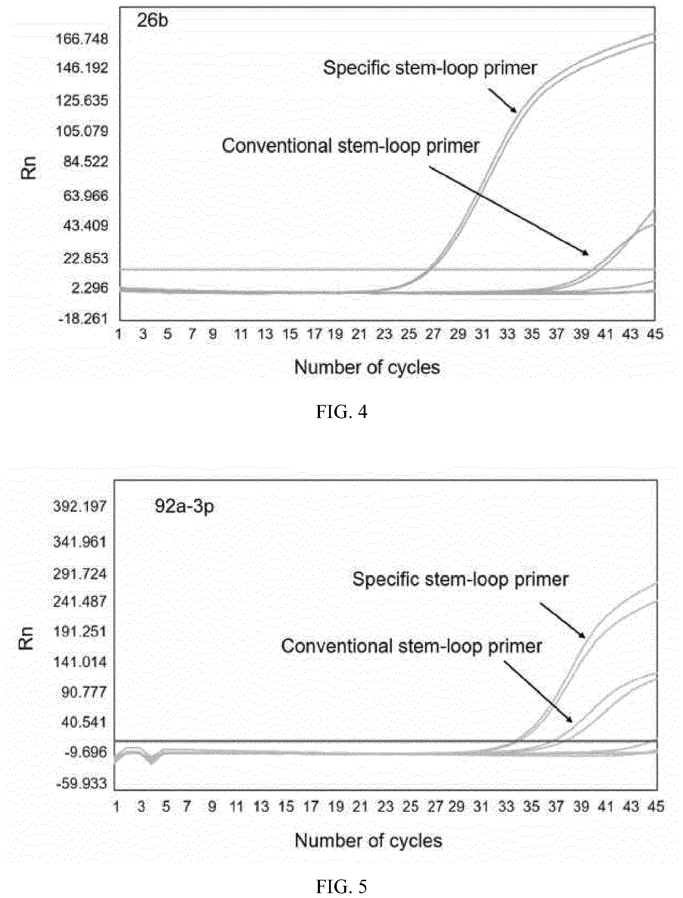

The four miRNAs in Table 1 were detected by using the primers and probes shown in Table 2 and Table 3, respectively. The amplification system is shown in Table 4, and the above reaction system was used to carry out real-time fluorescence quantitative PCR reaction on a SLAN96P fluorescence quantitative PCR instrument according to the amplification reaction procedure shown in Table 5. The experimental results are shown in Figs. 2-5 and Table 6. It can be seen from the results that, using the specific primers and probes designed by the method of the disclosure (that is, the improved specific stem-loop primers and probes), the Ct value of the one-step method amplification is much smaller than that of the conventional common stem-loop primers and probes, and NTC (no template) also has no non-specific amplification, and the fluorescence value is also higher than that of the conventional design. However, for conventional stem-loop primers and probes, except for delayed Ct value, NTC has non-specific amplification, which is not suitable for one-step fluorescence quantitative PCR system.

**Table 4: MicroRNA one-step amplification system**

| Reagent | Amount | Final concentration |
|---|---|---|
| 5× PCR Buffer for one-step method | 10 µL | 1× |
| 100 mM dNTP (U) | 2 µL | 4 mM |
| 1 M Mg²⁺ | 0.5 µL | 10 mM |
| RNasin | 0.125 µL | 10 U/µL |
| 5U hot start Taq enzyme | 3 µL | 0.3 U/µL |
| 5U reverse transcriptase | 2 µL | 0.2 U/µL |
| 1U thermosensitive UDG enzyme | 0.2 µL | 0.004 U/µL |
| Forward primer F (50 µM) | 0.3 µL | 0.3 µM |
| Reverse primer R (50 µM) | 0.3 µL | 0.3 µM |
| Probe P (50 µM) | 0.3 µL | 0.3 µM |
| Stem-loop reverse transcription primer RT (50 µM) | 0.1 µL | 0.1 µM |
| Template | 20 µL | |
| DEPC | Supplementing to 50µL | |

**Table 5: MicroRNA one-step amplification procedure**

| Temperature | Time | Number of cycles |
|---|---|---|
| 25°C | 10 min | 1 |
| 50°C | 10 min | 1 |
| 95°C | 3 min | 1 |
| 95°C | 15 s | 40 |
| 60°C | 30 s | |

**Table 6: Comparison of one-step amplification between specific stem-loop primers and conventional stem-loop primers**

| Template | Specific stem-loop primers (Ct) | Conventional stem-loop primers (Ct) |
|---|---|---|
| 106a | 29.24 | 35.06 |
| 106a | 29.83 | 35.65 |
| 106a NTC | No Ct | 39.17 |
| 106a NTC | No Ct | 39.57 |
| Let7a | 33.03 | 42.88 |
| Let7a | 33.06 | 44.15 |
| Let7a NTC | No Ct | 42.66 |
| Let7a NTC | No Ct | 44.11 |
| 26b | 26.33 | 39.87 |
| 26b | 26.55 | 40.45 |
| 26b NTC | No Ct | No Ct |
| 26b NTC | No Ct | No Ct |
| 92a-3p | 33.73 | 37.44 |
| 92a-3p | 33.39 | 36.45 |
| 92a-3p NTC | No Ct | 44.46 |
| 92a-3p NTC | No Ct | No Ct |

### Example 3: Sensitivity detection for miRNA one-step method

According to the primer and probe design of Example 1 and the one-step reaction system and procedure of Example 2, the sensitivity analysis of 106a was carried out as follows:

RNA-free water was added to Has-mir-106a dry powder (synthesized by Sangon Biotech Company) to dissolve into 10¹⁵ copies/mL, marked as E15, and then a series of solutions were diluted with RNA-free water according to a 10-fold concentration gradient, and E4 to E2 were selected as templates to perform sensitivity analysis of miRNA one-step method.

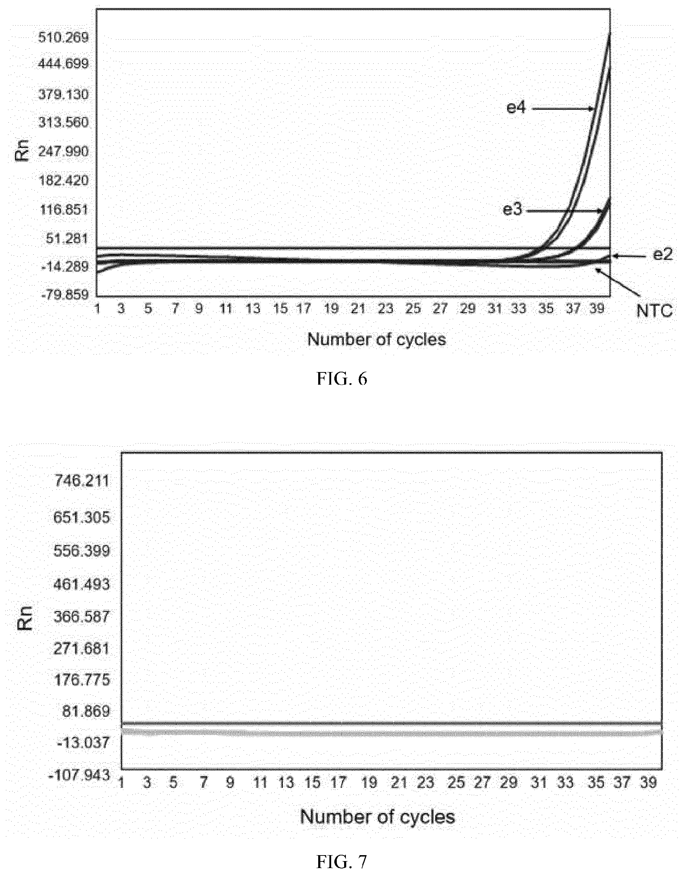

The experimental results are shown in Fig. 6 and Table 7. It can be seen that the amplified product has a typical S-shaped curve and a concentration gradient. The average Ct value of E4 is 34.84, the average Ct value of E3 is 37.41, and E2 is No Ct, NTC is No Ct, and the sensitivity reaches E3.

**Table 7: Sensitivity Analysis**

| Template concentration | Ct value |
|---|---|
| E4 | 34.62 |
| E4 | 35.05 |
| E3 | 37.49 |
| E3 | 37.32 |
| E2 | No Ct |
| E2 | No Ct |
| NTC | No Ct |
| NTC | No Ct |

### Example 4: Specificity detection for miRNA one-step method

The specificity analysis of primers and probes for Hsa-mir-92a was carried out as follows:
Hsa-mir-92a-3p (target sequence) and three interference sequences (Hsa-mir-106a, Hsa-mir-let7a, and Hsa-mir-26a sequences) were synthesized by Sangon Biotech Company. Using the primers and probes for Hsa-mir-92a designed in Example 1, the target sequence and the interference sequences were detected according to the method of Example 2, and the detection results are shown in Table 8 and Fig. 7. It can be seen from Table 8 and Fig. 7 that, the primers and probes only amplified Hsa-mir-92a, and did not amplify the other three interference sequences, indicating that the primers and probes involved in the disclosure have good specificity.

**Table 8: Specificity analysis**

| | Template concentration | Ct value |
|---|---|---|
| Target sequence | 92a-3p (10⁻⁶) | 31.91 |
| | 92a-3p (10⁻⁷) | 34.82 |
| | 92a-3p (10⁻⁸) | 37.37 |
| Interference sequence | 106a (E4) | No Ct |
| | Let7a (E4) | No Ct |
| | 26b(10⁻⁶) | No Ct |
| | NTC | No Ct |

### Example 5: Improvement of miRNA one-step system

In order to further improve the sensitivity of miRNA one-step detection, the inventors improved the amplification detection system and designed a new 5^{∗}PCR buffer for miRNA one-step detection. The components of the buffer include 150 mM Tris-HCl, 50 mM (NH₄)₂SO₄, 250 mM KCl, 0.5% Tween-20, 0.05% gelatin, 0.1% BSA, 10 mM EDTA, 5 M betaine, 10% sorbitol, 15% mannitol, 25 µg/µl gp32, 0.25 M TMAC, 3% glycerol and 10% PEG 6000. Further, in order to better illustrate the improvement effect, the inventor used a commercially available RT-PCR buffer for one-step amplification (Hifair^{®} III One Step RT-qPCR Probe Kit, catalog no. 11145ES50) from Yeasen Company for comparison.

The experimental results are shown in Table 9 below. Compared with the commercially available system, the miRNA one-step system of the disclosure has a more advanced Ct value and higher sensitivity. hsa-miR-106a detection had an average advancement of 4.3 Ct value, and hsa-miR-26b detection had an average advancement of 1.3 Ct value. In addition, the NTC fluorescence values in the results on the product of Yeasen Company were unstable, and the line shape was jittery, indicating that this system could not be fully adapted to the specific stem-loop primers and probes. In conclusion, the miRNA one-step system of the disclosure is more suitable for matching specific stem-loop primers and probes, and exhibits higher sensitivity and specificity in miRNA detection.

**Table 9: Comparative test of PCR one-step buffer and the existing one-step buffer**

| Template | One-step buffer of the disclosure (Ct) | One-step buffer of Yeasen Company (Ct) |
|---|---|---|
| 106a E5 | 28.05 | 32.60 |
| 106a E4 | 32.60 | 36.70 |
| 106a NTC | No Ct | No Ct |
| 26b 10⁻⁵ | 23.72 | 25.05 |
| 26b 10⁻⁶ | 27.55 | 28.75 |
| 26b NTC | No Ct | No Ct |

### Example 6: Influence of reverse primer design on one-step detection

Further, the inventors explored the influence of reverse primer design on the sensitivity of miRNA one-step detection. The inventors found that the range of reverse primers has a significant impact on sensitivity. Taking hsa-miR-106a as an example, the inventors designed a series of reverse primers as shown in Table 10 (the underlined and bold sequence is the starting sequence of the loop portion), and explored the Ct values.

**Table 10: Design of Reverse Primers**

| miRNA to be detected | Names of primers or probes | Nucleotide sequences 5'-3' | SEQ ID NO: |
|---|---|---|---|
| hsa-miR-106a | miR-106a-SL-Ra | TATCCGATC**GTAGCGTGGAGT** (9bp deviation to 5' end from the starting point of the circular sequence) | 41 |
| hsa-miR-106a | miR-106a-SL-Rb | CGATC**GTAGCGTGGAGTCG** (5bp deviation to 5' end from the starting point of the circular sequence) | 42 |
| hsa-miR-106a | miR-106a-SL-Rc | **CGTGGAGTCGGCAATT**GATC (4bp deviation to 3' end from the starting point of the circular sequence) | 43 |

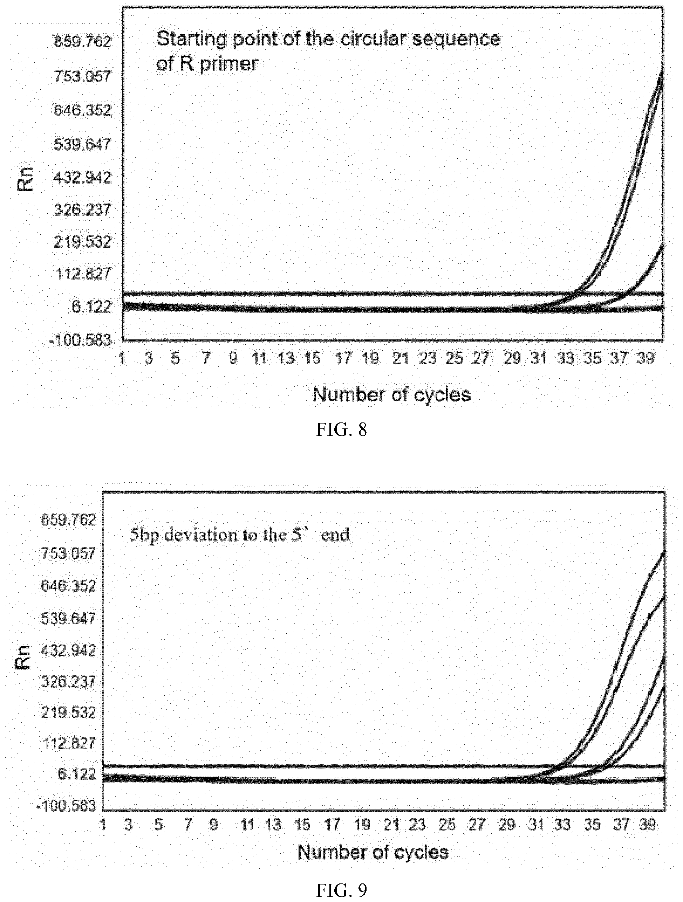

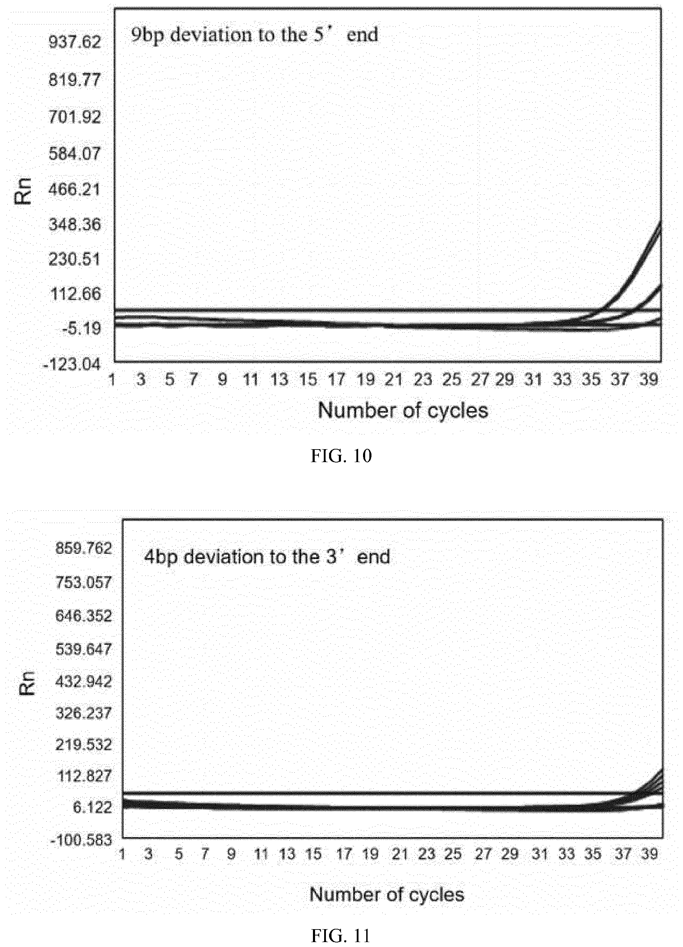

The experimental results are shown in Table 11 below and Figs. 8-11. The amplification was successfully performed by using R primer with the circular sequence as the starting point and primer sequence with 5 bp deviation to the 5' end from the starting point, wherein the lowest Ct value of e4 is 32.61, and the lowest Ct value of e3 is 35.51. Using a primer sequence with 9 bp deviation to the 5' end, or 4 bp deviation to the 3' end from the starting point of the circular sequence of R primer, the amplification efficiency was decreased, and the Ct value was delayed. To sum up, the starting point of the reverse primer is located at the starting point of the circular sequence of the stem-loop primer, or with a 1-8 bp deviation to the 5' end, or 1-3 bp deviation to the 3' end, and this design region has the best effect.

**Table 11: Region test for the design of reverse primers**

| Template | The starting point of circular sequence of R primer (Ct) | 5 bp deviation to the 5' end from the starting point of circular sequence of R primer (Ct) | 9 bp deviation to the 5' end from the starting point of circular sequence of R primer (Ct) | 4 bp deviation to the 3' end from the starting point of circular sequence of R primer (Ct) |
|---|---|---|---|---|
| 106a E4 | 33.52 | 32.61 | 35.76 | 39.23 |
| 106a E3 | 37.30 | 35.51 | 38.13 | 38.30 |
| 106a NTC | No Ct | No Ct | No Ct | No Ct |

### Example 7: Influence of forward primer design on one-step detection

Further, the inventors explored the influence of the design of forward primers on the sensitivity of miRNA one-step detection. Taking hsa-miR-106a as an example, the inventors designed primers and probes when N is 5-7 and N is 9 (the underlined and bold sequence is the complementary sequence of N), their sequences are shown in Table 12 below.

**Table 12: Design of forward primers**

| N=6 | | | |
|---|---|---|---|
| miRNA to be detected | Names of primers or probes | Nucleotide sequences 5'-3' | SEQ ID NO: |
| hsa-miR-106a | miR-106a-SL-F | GCGTCAAAAGTGCTTACAGTGC | 44 |
| hsa-miR-106a | miR-106a-SL-R | GTAGCGTGGAGTCGGCAATT | 45 |
| hsa-miR-106a | miR-106a-SL-P | ATCGGATACGAC**CTACCT** | 46 |
| hsa-miR-106a | miR-106a-SL-RT | GTCGTATCCGATCGTAGCGTGG AGTCGGCAATTGATCGGATACG ACCTACCT | 47 |

| N=5 | | | |
|---|---|---|---|
| hsa-miR-106a | miR-106a-L-F1 | CGTCAAAAGTGCTTACAGTGCA | 48 |
| hsa-miR-106a | miR-106a-L-R1 | GTAGCGTGGAGTCGGCAATT | 10 |
| hsa-miR-106a | miR-106a-L-P1 | ATCGGATACGAC**CTACC** | 49 |
| hsa-miR-106a | miR-106a-L-RT1 | GTCGTATCCGATCGTAGCGTGG AGTCGGCAATTGATCGGATACG ACCTACC | 50 |

| N=7 | | | |
|---|---|---|---|
| hsa-miR-106a | miR-106a-L-F2 | GGCGTCAAAAGTGCTTACAGTG | 51 |
| hsa-miR-106a | miR-106a-L-R2 | GTAGCGTGGAGTCGGCAATT | 10 |
| hsa-miR-106a | miR-106a-L-P2 | ATCGGATACGAC**CTACCTG** | 52 |
| hsa-miR-106a | miR-106a-L-RT2 | GTCGTATCCGATCGTAGCGTGG AGTCGGCAATTGATCGGATACG ACCTACCTG | 53 |

| N=9 | | | |
|---|---|---|---|
| hsa-miR-106a | miR-106a-L-F3 | CGGCGTCAAAAGTGCTTACAGT | 54 |
| hsa-miR-106a | miR-106a-L-R3 | GTAGCGTGGAGTCGGCAATT | 10 |
| hsa-miR-106a | miR-106a-L-P3 | ATCGGATACGAC**CTACCTGCA** | 55 |
| hsa-miR-106a | miR-106a-L-RT3 | GTCGTATCCGATCGTAGCGTGG AGTCGGCAATTGATCGGATACG ACCTACCTGCA | 56 |

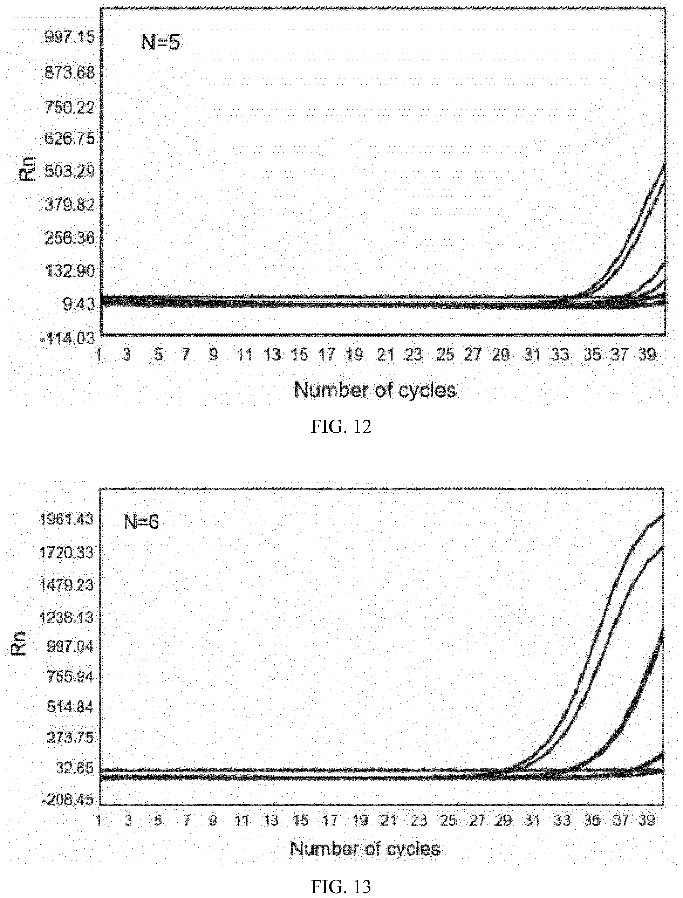

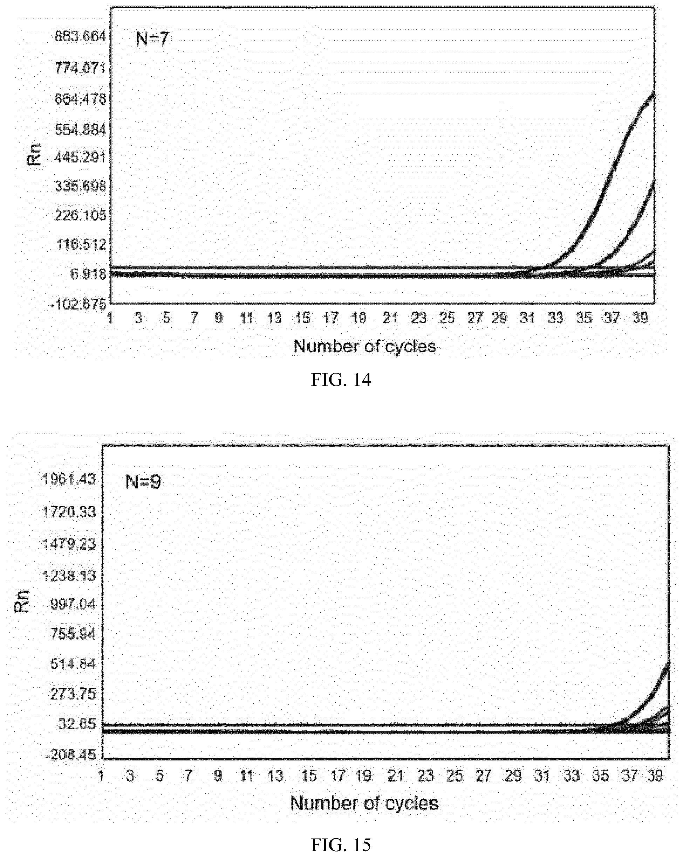

The experimental results are shown in Table 13 and Figs. 12-15. It can be seen from the results that, the specific primers and probes designed by the method of the disclosure are optimal when the number of N bases is 6. The target gene can be amplified normally by one-step method, and the Ct value is a concentration gradient, particularly the average Ct value of E5 is 29.38, the average Ct value of E4 is 33.43, and the average Ct value of E3 is 37.87. One-step amplification can be carried out when the number of N bases is 5 or 7. On the other hand, when the number of bases is 9, not only the amplification fails but also non-specific amplification occurs.

**Table 13: Comparison test on N base range**

| Template | Specific stem-loop primers, the number of N bases is 5 (Ct) | Specific stem-loop primers, the number of N bases is 6 (Ct) | Specific stem-loop primers, the number of N bases is 7 (Ct) | Specific stem-loop primers, the number of N bases is 9 (Ct) |
|---|---|---|---|---|
| 106a E5 | 33.64 | 29.03 | 31.98 | 37.81 |
| 106a E5 | 34.15 | 29.73 | 32.12 | No Ct |
| 106a E4 | 38.04 | 33.51 | 35.38 | 39.42 |
| 106a E4 | 37.06 | 33.34 | 35.22 | No Ct |
| 106a E3 | 39.31 | 38.00 | 38.96 | No Ct |
| 106a E3 | No Ct | 37.73 | 37.90 | 36.36 |
| 106a NTC | No Ct | No Ct | No Ct | 36.25 |
| 106a NTC | No Ct | No Ct | No Ct | 38.25 |

## Claims

1. A primer and probe design method for miRNA detection, comprising the following steps:
S1: designing a forward primer, wherein the sequence of the primer is the same as a sequence obtained by deleting the last N bases from the 3' end of a target miRNA and replacing U in the sequence with T so that the Tm value of the forward primer is 56 - 60°C;
S2: designing a stem-loop primer, wherein the forward primer is compared with a universal stem-loop primer to find out a region in which the number of consecutive bases of complementary pairing is greater than 5, then base substitution or deletion is conducted in the sequence of the universal stem-loop primer so that the number of consecutive bases of complementary pairing is less than or equal to 5, or the dG between the stem-loop primer and the forward primer is >-8kc/m;
S3: designing a reverse primer; and
S4: designing a probe;
wherein N is 5 - 8.

2. The primer and probe design method for miRNA detection according to claim 1, wherein the step S3 comprises making the reverse primer sequence identical to a part of the stem-loop primer, and the number of consecutive bases of complementary pairing for the stem-loop primer is less than or equal to 5 so that the Tm value of the reverse primer is 56 - 60°C; preferably, the starting point of the reverse primer is located at the starting point of the circular sequence of the stem-loop primer, or 1 - 8 bp deviation to the 5' end, or 1 - 3 bp deviation to the 3' end.

3. The primer and probe design method for miRNA detection according to claim 1, wherein the step S4 comprises making the probe sequence include two sequence segments F and G from the 5' end, the first sequence segment F is identical to the 3' end sequence of the stem-loop primer, and the second segment G is identical to the complementary sequence of the last N bases at the 3' end of the target miRNA, so that the Tm value of the probe is 68 - 72°C.

4. The primer and probe design method for miRNA detection according to any one of claims 1-3, wherein N is 6.

5. A miRNA detection composition comprising primers and probes designed by the method according to any one of claims 1-4.

6. The miRNA detection composition of claim 5, wherein each component of the composition is present in the same package.

7. Use of the miRNA detection composition according to claim 5 or 6 in the preparation of a kit for detecting miRNA.

8. A miRNA detection kit, comprising the primers and probes designed by the method according to any one of claims 1 - 4 or the miRNA detection composition according to claim 5 or 6.

9. The miRNA detection kit according to claim 8, wherein the kit further comprises a PCR amplification system.

10. The miRNA detection kit according to claim 8 or 9, wherein the kit further comprises 100 - 200 mM Tris-HCl, 30 - 100 mM (NH₄)₂SO₄, 200 - 300 mM KCl, 0.2 - 0.8% Tween-20, 0.02 - 0.08% gelatin, 0.05 - 0.2% BSA, 5 - 20 mM EDTA, 2 - 10 M betaine, 5 - 15% sorbitol, 5 - 20% mannitol, 15 - 30 µg/µl gp32, 0.2 - 0.3 M TMAC, 1 - 5% glycerol, and 5 - 15% PEG 6000.

11. The miRNA detection kit according to claim 8 or 9, wherein the kit further comprises at least one of nucleic acid preservation agent or nucleic acid release agent.

12. A method for one-step detection of miRNA, comprising the following steps:
1) releasing the nucleic acid of a sample to be tested;
2) detecting the nucleic acid obtained in the above step 1) by using the composition according to claim 5 or 6;
3) obtaining and analyzing the results.
